# EUROPEAN PATENT SPECIFICATION

(11) **EP 1 977 698 B1**
(45) Date of publication and mention of the grant of the patent: **09.12.2009**
(21) Application number: 07007118.8
(22) Date of filing: 04.04.2007
(51) Int. Cl.: A61B 17/04

(54) **Loading device for indwelling implement**
Ladevorrichtung für ein eingepflanztes Gerät
Dispositif de chargement pour engin à demeure

(43) Date of publication of application: 08.10.2008
(73) Proprietor: OLYMPUS MEDICAL SYSTEMS CORP., Shibuya-ku Tokyo 151-0072 (JP)
(72) Inventor: Hashimoto, Tatsutoshi, Shibuya-ku Tokyo (JP); Matsuno, Kiyotaka, Shibuya-ku Tokyo (JP)
(74) Representative: von Hellfeld, Axel

(56) References cited:
- WO-A-94/05213
- US-A1- 2003 216 613
- US-A1- 2005 149 067

## Description

### BACKGROUND OF THE INVENTION

### Field of the Invention

The present invention relates to a loading device for an indwelling implement that is employed to load a procedure tool into an applicator, the procedure tool (referred to as "indwelling implement" hereinafter) being of the type that is used to suture or tie off physiologic tissue, and then remains in the body thereafter.

### Description of the Related Art

Various methods are available for carrying out operative procedures on the internal organs of the human body, including laparotomy, in which an operative procedure is carried out by making a large incision in the abdominal wall; laparoscopic surgery in which the operative procedure is performed by approaching the abdominal cavity from an opening formed in the abdominal wall; and endoscopic techniques, in which the desired procedure is performed by inserting a flexible endoscope into the body via the mouth or anus.

In operative procedures employing these methods, such procedures as tissue suturing, tying off, and ligating are performer When carrying out such procedures, indwelling implements that can remain in the body are often used. Indwelling implements of this type are loaded into an applicator, and then placed in the desired location within the body by manipulating the applicator from outside the body.

However, the use of rubber gloves for operations makes it inconvenient to load a needle or thread a suturing material on a hook for loading an indwelling implement to an applicator. Suturing plural sites continuously will need a long operation time.

Document US 2005/0149067 A1, which corresponds to the preamble of the claim 1, concerns a loading device for an indwelling implement. In one embodiment, an end loop cartridge, an engagement female member and a removable needle used for a suturing device are held by an elastic member, such as rubber in a case. While the engagement female member is housed in the case, a free end portion of the engagement female member is preferably held by the elastic member such as rubber so as not to open.

Document WO 94/95213 concerns an endoscopic suture system. In one embodiment, a locking mechanism comprising resilient fingers for releasably retaining needles within a casing recess is disclosed.

### SUMMARY OF THE INVENTION

Accordingly, it is an object of the present invention to provide a loading device that easily loads an indwelling implement to an applicator in view of solving the above-described issues.

The loading device for an indwelling implement according to a first embodiment of the present invention loads an indwelling implement having a linear member into an applicator. The loading device for an indwelling implement includes the features of claim 1.

The loading device for an indwelling implement according to a second embodiment loads an indwelling implement for tightening down on tissues by moving a casing through which a linear member has been passed. The loading device for an indwelling implement includes a holder that holds the casing so as to enable pushing in of the casing; a biasing device that pulls the linear member which has been tied to the casing holder by the holder; and a guide part that engages with part of the applicator and causes an axis of the casing and an axis of a sheath that attaches to the casing to coincide.

The loading device for an indwelling implement according to a third embodiment loads an indwelling implement having a linear member into an applicator. The loading device for an indwelling implement includes: an end tip holder that holds a center tip that is provided at one end of the linear member that is freely attachable and releasable with respect to an end tip housing in the applicator, the end tip holder having a receiving part that guides the end tip housing of the applicator onto an end tip; an end tip locking member that comes into contact with the end tip inside the end tip holder so that the axis of the end tip and the axis of the receiving part roughly coincide, the end tip locking member being disposed so as to enable movement between a first position at which the movement of the end tip is restricted, and a second position which is removed from the movement path of the end tip housing when the end tip housing is attached to the end tip; a casing holder that is provided farther to the other end of the linear member than the end tip and holds a casing that houses the end tip, the casing holder holding the casing so that an axis of the casing holder and an axis of the casing attachment of the applicator which attaches the casing roughly coincide; a biasing device for pulling the linear member which is tied to the casing held by the casing holder; and a guide part which engages with part of the applicator and causes the axis of the casing and the axis of a sheath that attaches to the casing to coincide.

In accordance with the present invention, the loading device allows an indwelling implement to be easily loaded to an applicator. The same number of loading devices as sutures may be prepared and loaded into a suturing device, so as to shorten operation time and to reduce the stress of a patient.

### BRIEF DESCRIPTION OF THE DRAWINGS

FIG. 1 is a view showing the design of the tip of the applicator employed in this embodiment.
FIG. 2 is a view showing the design of the indwelling implement.
FIG. 3 is a cross-sectional view along a line A-A in FIG 2.
FIG. 4 is a view of the overall design of the loading device.
FIG. 5 is view along arrow B in FIG. 4.
FIG. 6 is a view in which the second base has been removed.
FIG. 7 is a view showing the needle locking member.
FIG. 8 is a view showing the first base.
FIG. 9 is a cross-sectional view long line C-C in FIG. 6.
FIG. 10 is a view for explaining the arrangement when the indwelling implement is housed in the loading device.
FIG. 11 is a view showing the structure of the winding cylinder.
FIG. 12 is a view showing the second base.
FIG. 13 is a view for explaining the operation for loading the indwelling implement into the applicator.
FIG. 14 is a partial view in cross-section depicting the state when the applicator has been pushed into the loading device.
FIG. 15 is a view in which the hook has been pushed in, thereby fastening the suture material to the hook.
FIG. 16 is a view in which the hook has been pulled back.
FIG. 17 is a view at the point when the holder has been opened from the state shown in FIG 9.
FIG. 18 is a view for explaining the process for using the indwelling implement to suture tissue, in which the curved needle of the applicator of the embodiments has been passed through tissue.
FIG. 19 is a view in which the forceps have been opened after the releasable needle of the indwelling implement has been stopped in the casing.
FIG. 20 is a view in which the indwelling implement is retained after suturing tissue.
FIG. 21 is a view of another embodiment in which the suture material is wound around a casing
FIG. 22 is a view showing the disposition of the indwelling implement in the arrangement shown in FIG. 18.
FIG. 23 is a view of another embodiment in which the suture material is wound around the casing.
FIG. 24 is a view for explaining the winding direction for the suture material.
FIG. 25 is a view showing the disposition of the indwelling implement in the arrangement shown in FIG. 20.

### DETAILED DESCRIPTION OF THE INVENTION

A description follows of a first embodiment of the present invention.

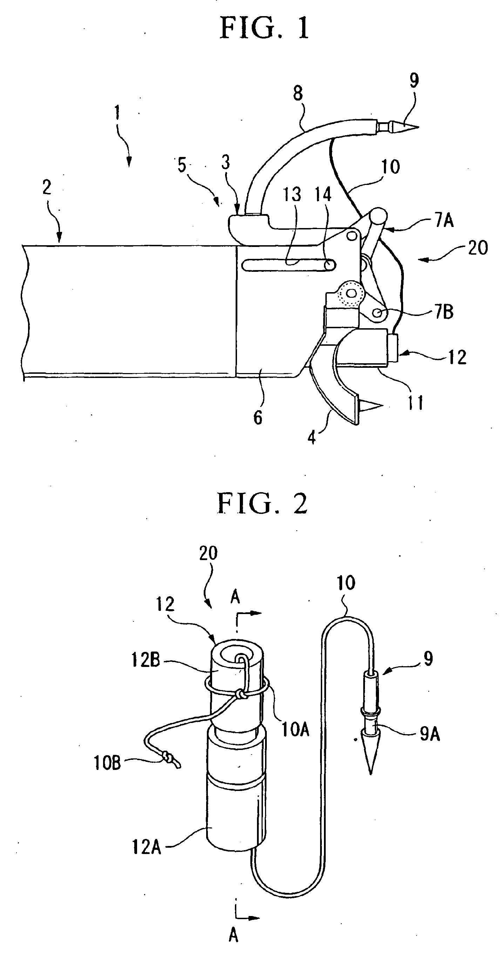

FIG. 1 shows a suturing device as one example of an applicator in this embodiment. A suturing device 1 has a pliable sheath 2 which is inserted into the body along with an endoscope. An operating unit (not shown), which is manipulated by the operator, is provided to the base part of sheath 2. A procedure part 5 that has a pair of openable and closable forceps members 3, 4 is provided to the end of sheath 2. The forceps members 3, 4 are supported on an end cover 6 to enable their free rotation, and are connected to a link mechanism 7. An operator wire, not shown in the figures, is advanced or retracted to drive the link mechanism 7, and thereby open or close the forceps members 3, 4. A curved needle 8 is provided to the end of forceps member 3, with a releasable needle 9, also referred to as the front tip, attached to the end of curved needle 8 in a freely releasable manner. A suture material 10, which is a linear member (alternatively, also referred to as "filament member"), extends from the base of the releasable needle 9. The suture material 10 consists of a monofilament, such as Nylon (registered trademark), for example. The suture material 10 is pulled out through a slit at the tip of the curved needle 8 and is pulled into a casing 12 which is attached to a hook sheath 11 (casing attachment) that can be freely advanced and retracted with respect to the top cover 6. The casing 12 is disposed on the axial line of the releasable needle 9 when paired forceps members 3, 4 are closed. By advancing the casing 12, the releasable needle 9 can be housed inside the casing 12. Note that a guide groove 13 is formed to the end cover 6 along the direction of insertion into the body. A pin 14 that is advanced and retracted along with the operating wire when the link mechanism 7 is driven is inserted into the guide groove 13.

The releasable needle 9 and the casing 12, which are connected by the suture material 10, form an indwelling implement 20 which is retained inside the body and is freely releasable with respect to the suturing device 1. In this indwelling implement 20, as shown in FIG. 2, the suture material 10, which extends from the base of the releasable needle 9, is pulled in through the end part 12A of the casing 12 and out through the opening in the base part 12B. Note that in this embodiment, the suture material 10 is fastened to the base part 12B by a single slip knot 10A. A knot 10B is formed at the other end of suture material 10.

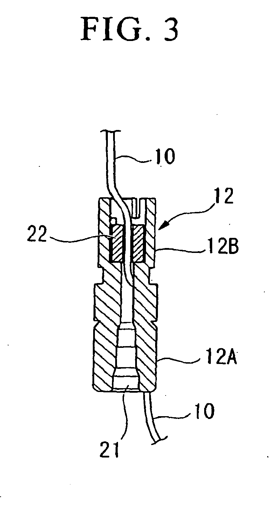

As shown in FIG 3, a hole 21 which can house the releasable needle 9 is present at the end part 12A side of the casing 12. A stop spring (not shown) which engages with a constricted part 9A (see FIG 2) of the releasable needle 9 and prevents the releasable needle 9 from being pulled out is inserted into the hole 21. The suture material 10 is pulled in so as to avoid the opening of the hole 21. Pressure is applied on brake parts 22, which are a combination of an elastic member and a rigid member after which the suture material 10 is pulled out. The brake parts 22 are housed in the casing 12 and are for applying a specific resistance on the suture material 10 after the end of the suture material 10 that is between brake members 22 has been pulled to tighten and tie down the functional portion of the indwelling implement 20 on the tissue which is the site (target site) for performing an operation such as suturing, tying down or ligating, so that this tied down state is not easily released.

Note that for convenience in the following description, the region on the releasable needle 9 side interposed between the brake members 22 (or within casing 12 housing the brake members 22) is referred to as the "procedure side," (i.e., the site where the procedure is performed on the tissue), and the region on the opposite side is referred to as the "non-procedure side" (i.e., the side manipulated by the applicator in order to carry out the procedure).

Next, a description will be made of a loading device used when loading the indwelling implement 20 into the suturing device 1, which is one example of an applicator.

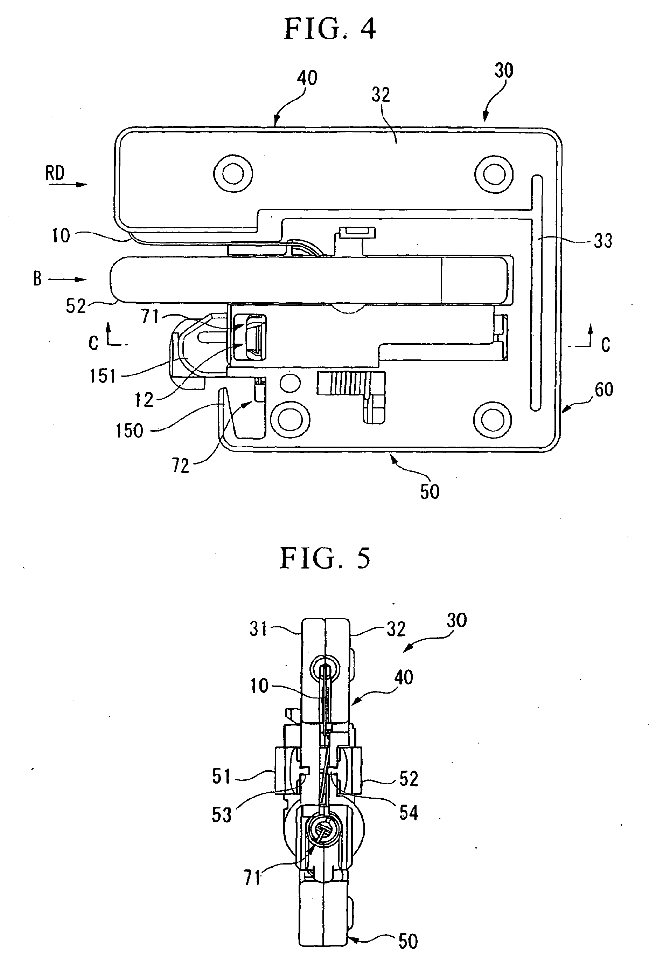

A loading device 30 has an external appearance such as shown in FIGS. 4 and 5. This loading device 30 is formed by stacking together a first base 31 and a second base 32 which consist of resin molded articles. Due to the presence of a roughly T-shaped slit 33, the loading device 30 comprises a long narrow needle holder 40 (end tip holder), a roughly square casing holder 50 that houses a casing 12 provided with the brake members 22, and an arm part 60 that extends from the corner of the casing holder 50 and connects to needle holder 40. A pair of locking members 51, 52 is attached in a freely openable and closable manner to the casing holder 50. Projections 53, 54 are formed facing one another at the edge of locking members 51, 52. Note that in this design, the suturing device 1 (applicator) shown in FIG 1 is introduced into the loading device 30 from the direction indicated by an arrow RD, and the indwelling implement 20 is then attached to the suturing device 1. In the following description of the loading device 30, the end farther away from the receiving direction is designated as the base, and the end closer to the receiving direction is designated as the front.

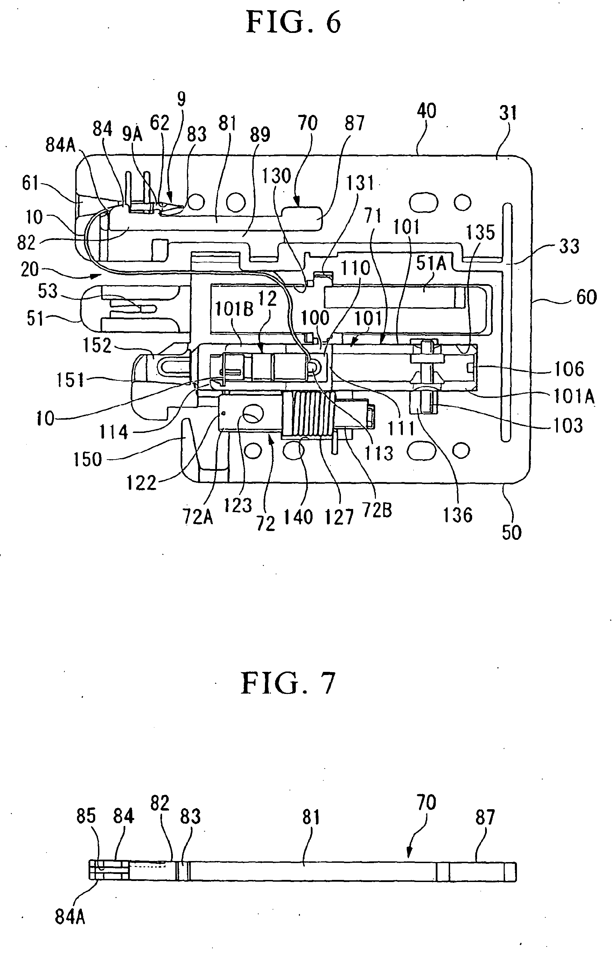

FIG 6 shows the state when the second base 32 has been removed. A concave receiving part 61 having a surface that tapers toward the end is provided to the needle holder 40 of the first base 31. A housing groove 62 for housing the releasable needle 9 is formed at the end of the receiving part 61. The movement of the releasable needle 9 when housed in housing groove 62 is restricted by pressing down on a needle locking member 70 (front tip locking member). As shown in FIGS. 6 and 7, the needle locking member 70 has a narrow long main part 81, and two convex parts 83 and 84 that are provided at the end part 82. The convex part 83 comes into contact with the constricted part 9A of releasable needle 9. A slit 85 is provided to the convex part 84. By passing the suture material 10 through the slit 85, the axes of the releasable needle 9 and the suture material 10 can be made to coincide. Note, that in order to facilitate pulling out of the suture material 10, receiving part 61 is provided with a cut-out part 63 formed on the casing holder 50 side.

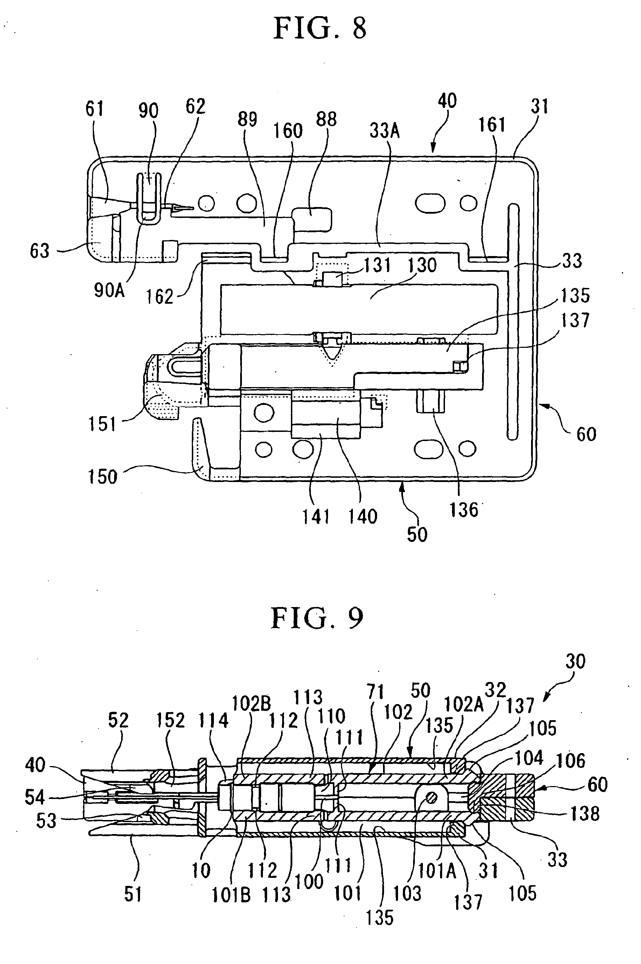

As shown by the arrangement in FIG 8, in which the needle locking member 70 has been removed, the area in which the needle locking member 70 is disposed forms a concave part 89 that comprises a large cut-out that excludes supporting part 88, corresponding to the area where base part 87 is disposed. Accordingly, the needle locking member 70 is cantilevered by needle holder 40 at base part 87. Note that a stopper 90 is provided to first base 31 so that the needle locking member 70 does not bend. The stopper 90 consists of an elastic piece which is biased in a direction that corrects bending in the needle locking member 70. In the starting state, an end part 90A comes into contact with the needle locking member 70. The position of the needle locking member 70 corresponds to a first position in which the movement of the releasable needle 9 is restricted.

As shown in FIG. 6, a suture material 10, which has been pulled out from the needle holder 40, is then pulled into casing holder 50. This casing holder 50 is provided with a holder 71 and a winding cylinder 72 which are disposed in parallel and in sequence from the locking member 51 side of the casing holder 50. The suture material 10 is pulled into the holder 71 through a slit 100 formed in the side of the holder 71. A casing 12 is housed inside the holder 71 The base part 12B of the casing 12 is partially exposed outside of the holder 71, and the suture material 10 that is tied to this base part 12B is also exposed outside of the holder 71.

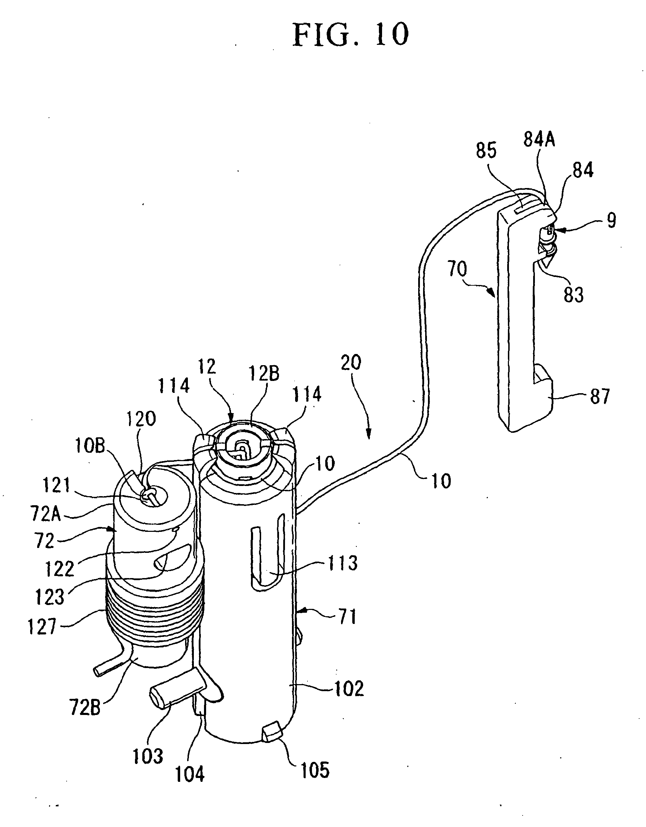

As shown in FIGS. 6, 9 and 10, the holder 71 has holder pieces 101,102 that are formed by dividing a cylinder into two parts. The holder pieces 101,102 are connected at the area around the base parts 101A, 102A by a pin 103. A space 104 is formed by introducing a cutout further toward the base side of the holder 71 than the pin 103. The end parts 101B, 102B can be opened, bringing the base parts 101A, 102A closer together. Respective convex parts 105 (controller for opening and closing) are formed to each of the holder pieces 101, 102 projecting outward from the base parts 101A, 102A side. A concave part 106 is formed to the inside of base part 101A of the one holder piece 101, at a position that is further toward the base side of the holder 71 then the convex part 105.

A holder 110 that holds the casing 12 is formed between the respective end parts 101B, 102B of the holder pieces 101, 102. The base of the holder 110 is partitioned by a stopper 111. The movement of the casing 12 within holder 110 is restricted by the stopper 111 and a more forwardly formed convex part 112. In the starting state, the end part 12A of the casing 12 is engaged and stopped by an elastic convex part 113 which is formed in a unitary manner with holder pieces 101, 102. At this time, the base part 12B of the casing 12 projects out by just a specific length from end parts 101B, 102B of the holder 71. The non-procedure region of the suture material 10 is wound around this outward projecting part. This wound part (hereinafter, also referred to for convenience as "traction part") is caught on a hook 15 of the suturing device 1, which will be described below. Note that while the base part 12B of the casing 12 is exposed outside the holder 71 in the starting state, the distance from the stopper 111 to the end parts 101B, 102B of the holder pieces 101, 102 is longer than the casing 12, so that the entire casing 12 can be pushed into the holder 71.

Respective claws 114 are provided extending out from the end parts 101B, 102B of each of the holder pieces 101, 102. The claws 114 are designed to extend out so as to avoid the suture material 10 and come into contact with the base part 12B of the casing 12, such that suture material 10 which is wound around the outer periphery of the casing 12 does not fall.

The end of the suture material 10 which has been pulled out from the casing 12 (i.e., the non-procedure end) is engaged and stopped in place on a winding cylinder 72. Winding cylinder 72 is a cylindrical body forming a biasing device. In winding cylinder 72, as shown in FIGS. 6, 10, and 11, a groove 120 is formed to the end part 72A directed toward the center of the cylinder. This groove 120 is of a width that enables passage of the suture material 10, and suture material 10 has a width that enables just one strand to pass at the area nearest center part 121. The center part 12 is wider in width than the groove 120, and the knot 10A is housed there. A hole 122 is provided to the side of the end part 72A at a position opposite groove 120. The diameter of the hole 122 is large enough for one strand of the suture material 10 to pass through. Penetrating hole 123 is formed to the end part 72A passing through the radial direction. A convex part 125 and a slit 126 are provided to the side of the base 72B of the winding cylinder 72. One end of a torsion coil spring 127, which is a biasing member composing a biasing device, is engaged and stopped in the slit 126. Torsion coil spring 127 generates twisting stress on winding cylinder 72, to apply a desired rotational force on the winding cylinder 72.

As shown in FIGS. 6 and 8, an opening 130, long and narrow in the receiving direction, for supporting locking member 51 is formed to first base 31. A concave groove 131 for supporting locking member 51 is provided perpendicular to the receiving direction along opening 130. In addition, a groove 135, long and narrow in the receiving direction, for housing the holder 71 is formed parallel to opening 130. A long groove 136 for supporting pin 103 of the holder 71 extends in a direction perpendicular to the receiving direction along a groove 135. A width of the long groove 136 in the receiving direction is larger than the outer diameter of the pin 103, and enables sliding of the pin 103 in the receiving direction. In addition, a convex part 137 is provided to the base part of the groove 135. As shown in FIG. 9, in the starting state, a convex part 137 is further toward the front than convex part 105 at the base of the holder 71.

As shown in FIGS. 6 and 8, a groove 140 for housing winding cylinder 72 is formed adjacent to the groove 135. A stepped part 141 is provided to groove 140, and enables stopping of the convex part 125 of winding cylinder 72. An elastic deformable claw 150 is provided in a unitary manner from the corner of the casing holder 50 on its groove 140 side. A claw 150 extends toward a receiving part 151, the end of which projects out from the casing holder 50, and forms a specific clearance together with the receiving part 151. The end surface of receiving part 151 is curved to match the profile of forceps member 4. A guide cylinder 152 (guide part) is formed to the receiving part 151 as shown in FIG 9. The outer diameter of the guide cylinder 152 is roughly equal to the outer diameter of the hook sheath 11 (casing attaching part) on the suturing device I side shown in FIG 1. The center axis of the guide cylinder 152 coincides with the axis of the casing 12 which is held by the holder 71.

Slit 33 which is formed in first base 31 has a curved part 33A that partitions the needle holder 40 and the casing holder 50. The needle holder 40 and the casing holder 50 are alternately evaginated along the receiving direction. Convex parts 160,161 which serve as restricting members are provided to the evaginated part on the needle holder 40 side, and a concave part 162 which serves as a restricting member is formed to the evaginated part on the casing holder 50 side. These convex parts 160,161 and concave part 162 are disposed on a straight line in the receiving direction.

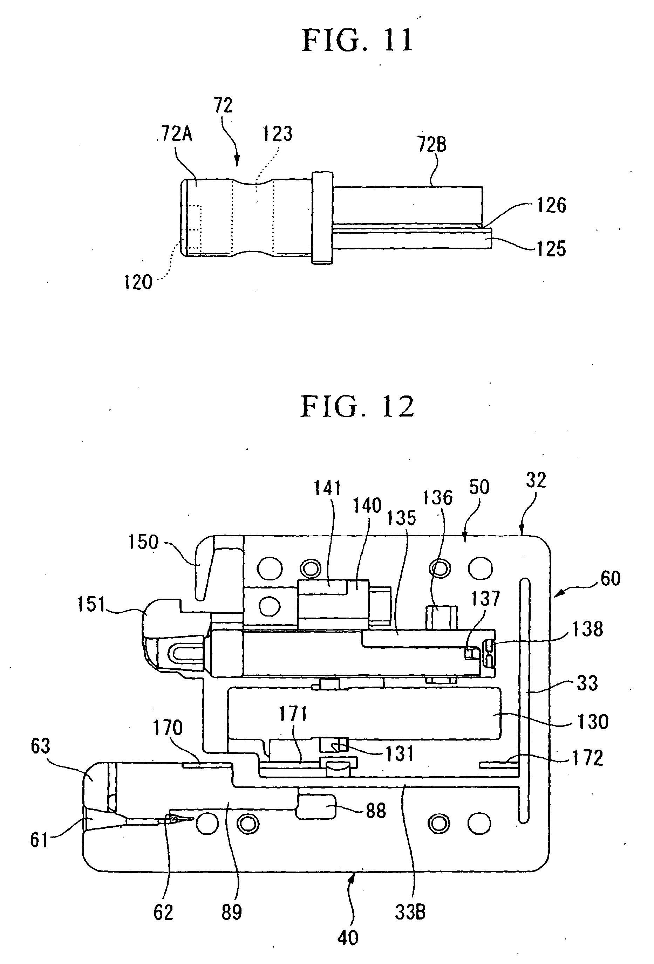

As shown in FIG 12, the second base 32 is roughly symmetrical in shape to the first base 31. The needle holder 40 is not provided with a stopper 90 such as shown in FIG 8, however. A convex part 138 is provided to the groove 135 for housing the holder 71 in the casing holder 50, further toward the base end side than the convex part 137. In the starting state shown in FIG 9, the convex part 138 engages with the concave part 106 (controller for opening and closing) of the base part 101A of the holder piece 101 of the holder 71. Further, the slit 33 has a curved part 33B that partitions the needle holder 40 and the casing holder 50. The needle holder 40 and the casing holder 50 are alternately evaginated along the receiving direction. A convex part 170 which serves as a restricting member is provided upright to the evagainated part on the needle holder 40 side, and the concave parts 171,172 which serve as restricting members are formed to the evaginated part on the casing holder 50 side. The convex part 170 and concave parts 171 and 172 are disposed along a straight line in the receiving direction. The convex part 170, the concave part 171, and the concave part 172 engage with the concave part 162, the convex part 160, and the convex part 161 of the first base 31, respectively.

Next, the process for attaching the indwelling implement 20 in the loading device 30 will be described.

The releasable needle 9 (front tip) is introduced into the housing groove 62 of the needle holder 40 shown in FIG. 6. The needle locking member 70 comes into contact with the constricted part 9A of the releasable needle 9, so that the position of the releasable needle 9 is held and the needle is prevented from falling out. The suture material 10 which extends from the releasable needle 9 is pulled out by passing through the slit 85 of the needle locking member 70, and pulled into the casing holder 50 by passing through a cut-out 63 in the receiving part 61. The suture material 10 passes between the locking members 51, 52, and is inserted into the holder 71 through the groove 10 on the side of the holder. The casing 12 through which the suture material 10 passes is housed in the housing 110 of the holder 71. The base part 12B of the casing 12 is exposed from the front of the holder 71 at this time. After the winding suture material 10 around the portion that is exposed from the holder 71, the suture material 10 is pulled out from the holder 71 toward the winding cylinder 72. The knot 10B at the end of the suture material 10 is housed in the center part 121 of the front of the winding cylinder 72, and the suture material 10 is caught in the groove 120. The winding cylinder 72 twists a coil torsion spring 127 and is mounted in bases 31, 32. As a result, tension is applied on the part of the suture material 10 that is wound around the casing 12 (also referred to as "loop portion" hereinafter), causing a reduction in the inner diameter. The bases 31, 33 are stopped by a screw or the like, thereby completing the attachment of the indwelling implement 20.

The process for attaching the indwelling implement 20 in the suturing device 1 (applicator) using the loading device 30 will now be described.

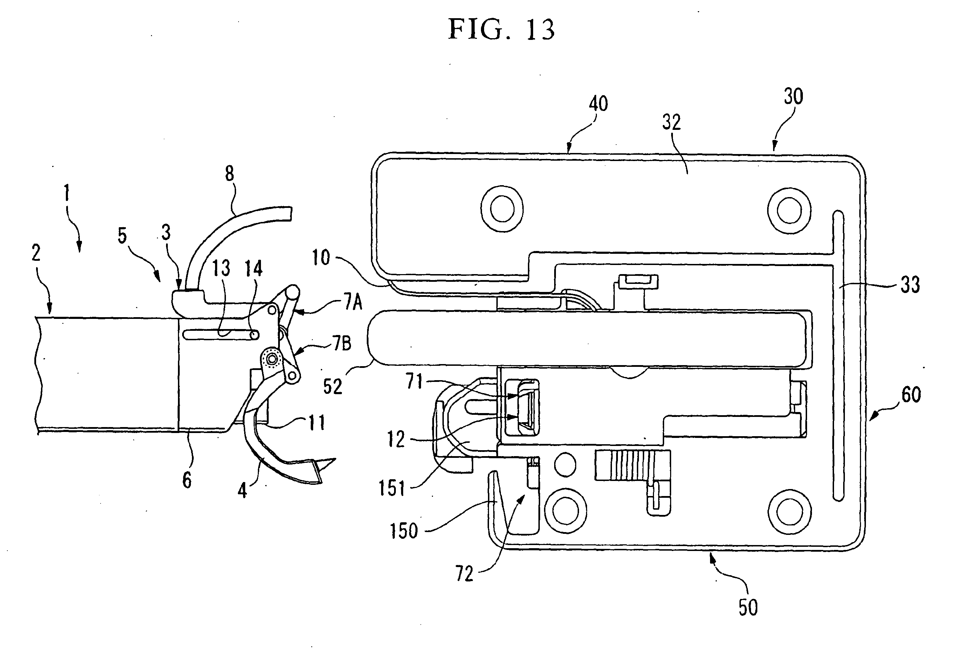

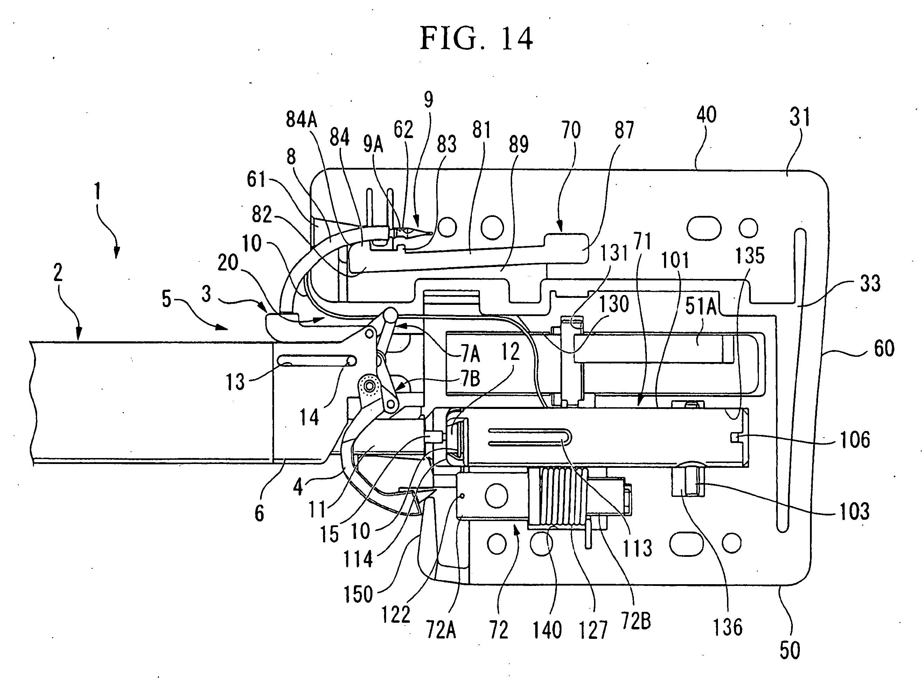

With the forceps members 3, 4 in the completely open state, the suturing device 1 is pushed into the loading device 30, as shown in FIG. 13. Then, as shown in FIG. 14, the projection 54 of one of the locking members 52 engages in a guide groove 13 in the end cover 6 of the suturing device 1. The projection 53 of the other locking member 51 advances into a guide groove 13 that is on the opposite side of the end cover 6, pushes down on the pin 14, and thereby maintains the forceps members 3, 4 in the open state. Note that projections 53, 54 of the locking members 51, 52 have an inclined surface that has a small slope angle in the receiving direction. As a result, when the suturing device 1 is inserted, these projections 53, 54 act to push open the locking members 51, 52. Further, since the projections 53, 54 have a large slope angle in the direction opposite the receiving direction, the engagement cannot be released simply by pulling on the suturing device 1. Moreover, the locking members 51, 52 are biased by a flat spring 51A at their base end side so as to close the projections 53, 54 sides. As a result, a state of engagement is formed between suturing device 1 and loading device 30 via these locking members 51, 52.

The curved needle 8 of the forceps member 3 is introduced into the receiving part 61 of the needle holder 40 at this time. The receiving part 61 is in the shape of a truncated cone such that the diameter of the opening becomes smaller moving progressively deeper into the device. As a result, even if there is a very small deviation between the curved needle 8 (end tip housing) and the receiving part 61, the curved needle 8 is guided to the releasable needle 9 in the housing groove 62 by following the taper of the receiving part 61. The curved needle 8 comes into contact with the slanted surface 84A of the end 84 of the needle locking member 70, and begins to engage with releasable needle 9 while pushing away the needle locking member 70. The needle locking member 70 which has been pushed away by the curved needle 8 moves past stopper 90, with its cantilevered base part 87 serving as a fulcrum. At this time, the needle locking member 70 is in the second position which is withdrawn from the path of movement of the curved needle 8.

The end part 90A of the stopper 90 enters into the concave part 89 once the needle locking member 70 is removed. As a result, even if the needle locking member 70 attempts to return to its original position, it cannot because the end part 90A of the stopper 90 interferes. Accordingly, the curved needle 8 is not interfered with by the needle locking member 70, and can therefore engage with the releasable needle 9. In this case, the suture material 10 which is centered at the needle locking member 70 is introduced into the slit formed in the curved needle 8.

The releasable needle 9 is supported slightly around the front, so that the curved needle 8 engages in the releasable needle 9 while pushing the needle holder 40 in the receiving direction. The needle holder 40 can move freely with respect to casing holder 50 trough the elastic deformation of arm 60. However, the movement of the needle holder 40 is restricted in the receiving direction only, by the restricting members formed through engagement between the convex parts 160, 161 and 170 on the needle holder 40 side and the concave parts 163, 171 and 172 on the casing holder 50 side. Accordingly, secure attachment can be accomplished without deviation between the axial line of the end of the curved needle 8 and the axial line of the releasable needle 9.

Meanwhile, the forceps member 4 comes into contact with the casing holder 50 so as to lie along the curved profile of the end surface of the receiving part 151. In addition, the front part of forceps member 4 presses the claw 150. When the claw 150 is pushed by the forceps member 4, the forceps member 4 is biased in the opening direction by the repelling force. As a result, even if there is a manufacturing error or play in the forceps member 4, the paired forceps members 3, 4 are forcibly opened to a state suitable for attachment of the indwelling implement 20.

Next, when a hook sheath 11 (i.e., a sheath for housing a traction member for pulling the non-procedure side of the indwelling implement 20) is advanced and inserted into the guide cylinder 152 formed in the receiving part 151, the front part of the hook sheath 11 is guided to the guide cylinder 152. As a result, the axial line of the hook sheath 11 and the axial line of the casing 12 coincide. A hook 15 within the hook sheath 11 is then advanced. The hook 15 is introduced into the casing 12, and bumps into the brake part 22 (see FIG 3). When the hook 15 is advanced further, the casing 12 is rushed via the brake part 22 into the holder 71. The casing 12 passes the elastic convex part 113, and moves to the point where it comes into contact with the stopper 111 of the holder 71. As a result, the casing 12 is pushed into the holder 71.

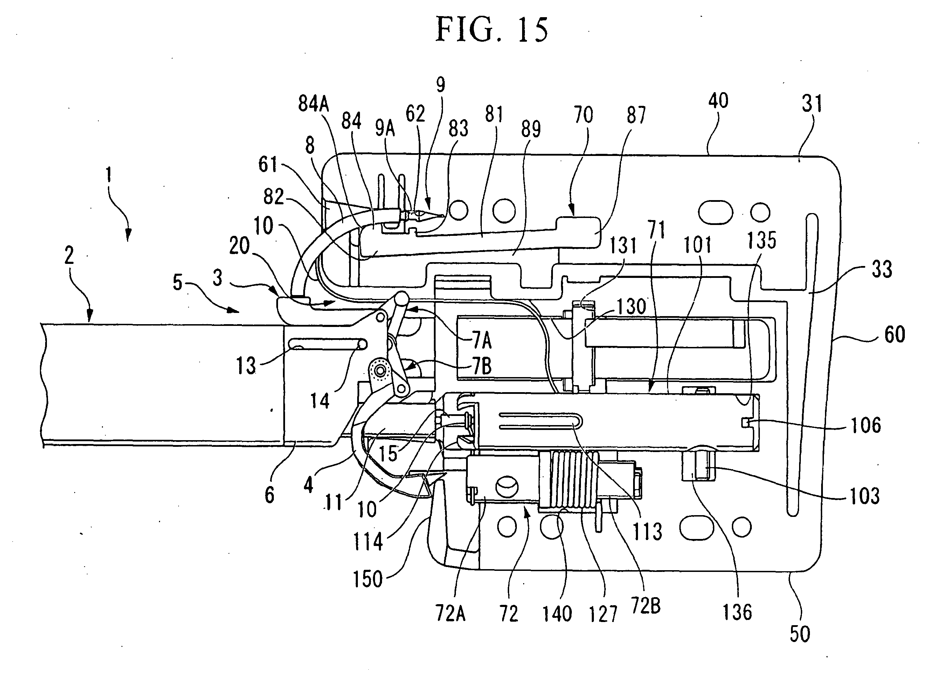

The end opening of the holder 71 has a diameter that is roughly equal to the casing 12, so that the suture material 10 which is wrapped around the casing 12 remains without being pulled into the holder 71. The suture material 10 is biased in the tightening direction by the spiral coil spring 127 of the winding cylinder 72. As a result, by pushing in the casing 12, the suture material 10 can be pulled to rotate the winding cylinder 72 and decrease the inner diameter of portion (loop portion) 10A which is the wound part of the suture material 10. As a result, as shown in FIG 15, the wound portion 10A of the suture material 10 is tightened on the trunk portion of hook 15.

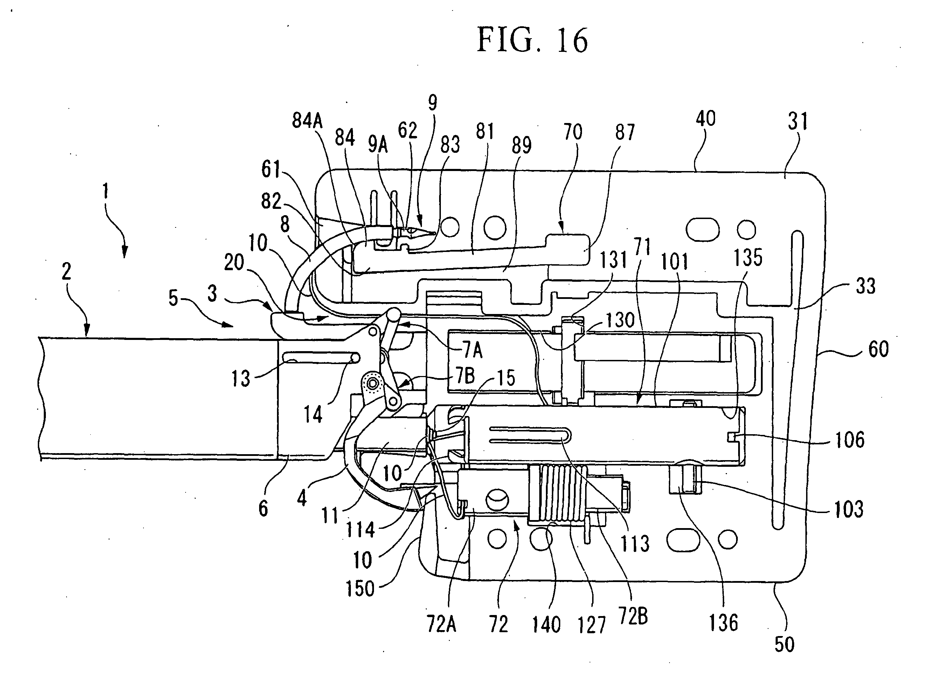

Once the suture material 10 is attached to the hook 15, the hook 15 is pulled back. As shown in FIG 16, the wound portion 10A (also referred to as "loop portion" below) of the suture material 10 that is in the non-procedure region and is interposed between the brake members 22 of the indwelling implement 20 is pulled into hook sheath 11 along with hook 15. The groove 120 and the center part 121 of the winding cylinder 72 can hold the suture material 10 in the radial direction. However, the force holding suture material 10 in the receiving direction is weak. As a result, when the amount of the suture material 10 pulled in exceeds the height of the groove 120 of winding cylinder 72, the suture material 10 slips out from winding cylinder 72.

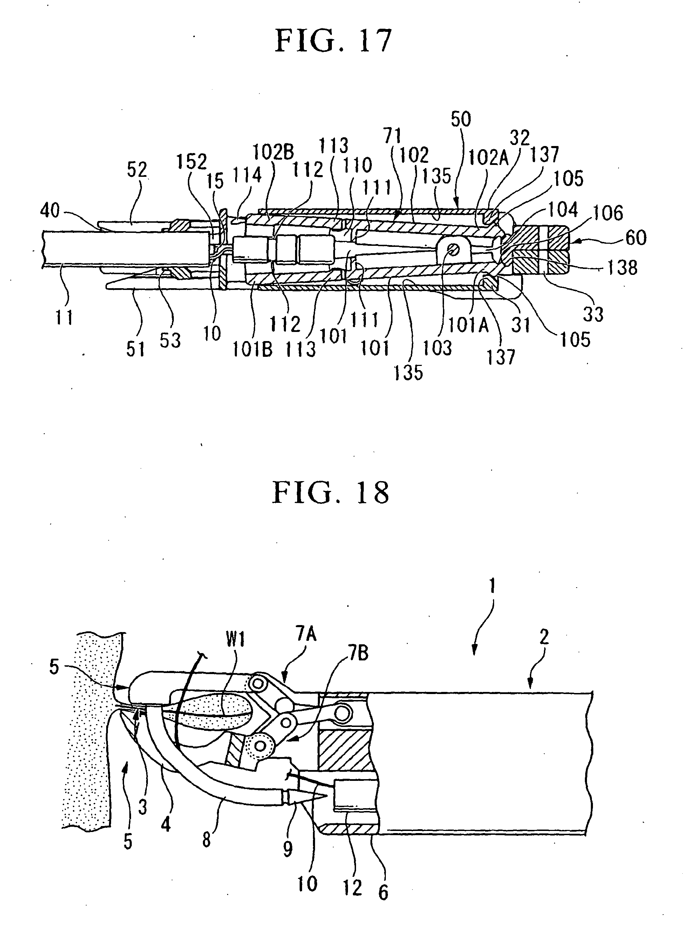

Further, when hook 15 is pulled back, this results in pulling by the suture material 10, with the casing 12 being pulled in the direction that pulls it out from the holder 71. As shown in FIG. 9, the holder 71 engages with casing 12 at convex part 112, so that it is pulled along with casing 12. In the starting state, the concave part 106 on the base side and the convex part 138 of second base 32 engage, so that the holder 71 does not move. However, when the pulling force becomes large, the engagement with concave part 106 is released. As a result, the holder 71 is able to slide in the pull-out direction by just the length of long groove 136 (see FIG 6). Further, during the process in which the holder 71 slides in the pull-out direction, the convex part 105 of the holder 71 and the convex part 137 of bases 31, 32 come into contact, and the base parts 101A, 102A of holder pieces 101,102 are pushed so as to approach one another, with holder pieces 101,102 rotating around the pin 103 as an axis. As a result, as shown in FIG 17, end parts 101B, 102B of holder pieces 101,102 open. Since the engagement with casing 12 is released, casing 12 can be pulled out from the holder 71. By then pulling the hook 15, the casing 12 comes into contact with the end part of hook sheath 11.

When the hook sheath 11 is pulled in, and the casing 12 is housed in end cover 6, the front is opened by pushing the base part of locking members 51,52 of the loading device 30 with the finger. As a result, the engagement is released between the projections 53, 54 of the locking members 51,52 and the guide grooves 13 of end cover 6. The loading device 30 and the suturing device 1 separate due to the elastic force of the arm 60 which returns the needle holder 40 and the elastic force of the claw 150 of the casing holder 50, so that the indwelling implement 20 is in the loaded state shown in FIG 1.

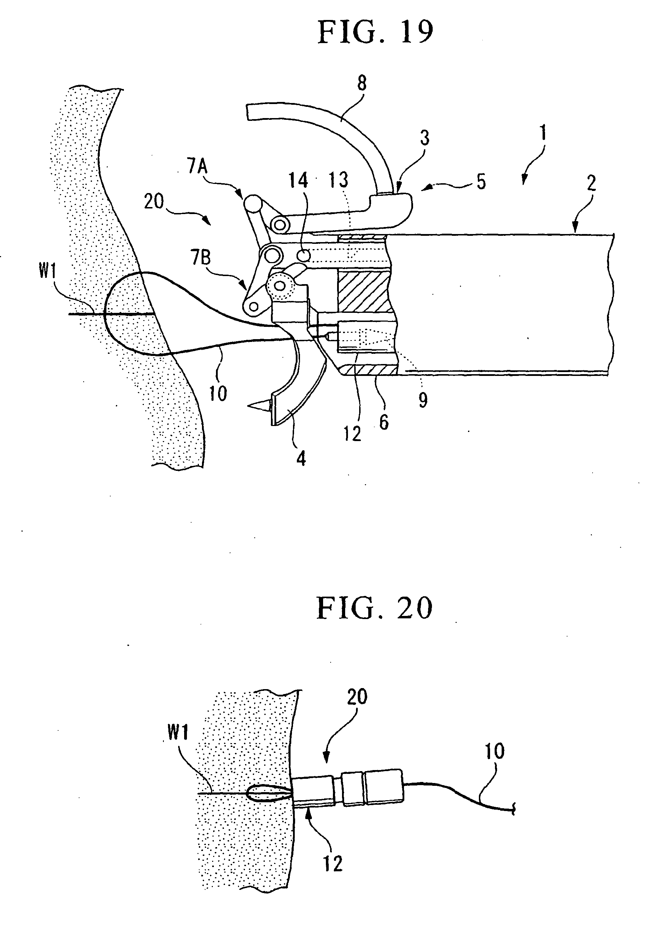

Note that when the indwelling implement 20 is used to suture close an opening in the tissue, the pair of forceps members 3, 4 face the opening to be sutured with the forceps in the open state. Next, the suturing device 1 is manipulated to close the pair of forceps members 3,4. As shown in FIG. 18, the tissue on one side of the area around opening W1 is gathered together with the forceps members 3,4, and the curved needle 8 is passed through the tissue from the other side, crossing opening W1. Next, the hook sheath 11 is advanced, the releasable needle 9 is housed inside the casing 12, and an engagement is made between the releasable needle 9 and the casing 12. When the casing 12 is subsequently retracted, the releasable needle 9 is freed from the curved needle 8. The forceps members 3,4 open as a result. As shown in FIG 19, the region on the procedure side of the suture material 10 is passed through the tissue to form a loop. As a result, when the hook 15 is withdrawn, traction is applied on the non-procedure side of the indwelling implement 20. As a result, the suture material 10 that is in the region on the procedure side is pulled and moves into the non-procedure side. As a result, the inner diameter of the loop formed in the region on the procedure side decreases. During this operation, tensile force is generated in the suture material 10, and the loop portion (wound part 10A) that is pulled by hook 15 does not readily become free from hook 15. When releasing the loop portion (wound part 10A) from hook 15 in order to leave the indwelling implement 20 inside the body, the hook 15 is moved in the direction that releases the tensile force, so that the indwelling implement 20 is freed from the loop portion that is caught on hook 15, and remains in the body.

Note that when the suture material 10 is formed of a material that slides well (monofilament Nylon in this embodiment), then, when the loop portion that is caught on the hook 15 is pulled into the non-procedure area by hook 15, the inner diameter of the loop opens easily under the force of traction. For this reason, when the indwelling implement 20 remains inside the body, the loop portion is even easier to free from hook 15. However, the present invention is not limited to this design. Rather, it is also acceptable to leave the indwelling implement 20 remaining after tying down the tissue by tightening the suture material 10, and then cutting the suture material 10. In addition, in the case where the position of the non-procedure region of the suture material 10 becomes longer after tying down the tissue, it is also acceptable to cut the suture material 10 to the desired length.

This type of the indwelling implement 20 remains inside the body with opening W1 in the tissue sutured closed, as shown in FIG 20.

In this embodiment, by positioning and holding the releasable needle 9 (end tip) and the casing 12, the releasable needle 9 can be loaded into the curved needle 8, and the axial line of the hook sheath 11 and the axial line of the casing 12 can be aligned simply by pushing on the suturing device 1. Further, when the casing 12 is pushed in with the hook 15, the suture material 10 that is wound on the casing 12 can be wound around the trunk portion of the hook 15. In particular, whereas previously operations such as loading the releasable needle 9 or threading the suturing material 10 on the hook 15 with rubber gloves worn for the procedure was difficult, such inconvenience is eliminated in this embodiment, so that anyone can easily load the indwelling implement 20. In particular, when suturing a number of continuous sites, a number of loading devices 30 corresponding to the number of sutures is prepared, and are sequentially loaded into the suturing device 1. Thus, the duration of the operation can be reduced and the stress on the patient lessened.

Further, since a biasing forces was applied to wind the suture material 10 around the torsion coil spring 127 as a means for tightening the suture material 10 on the hook 15, the loading device can be made smaller as compared to a design in which the suture material 10 is pulled in a straight line.

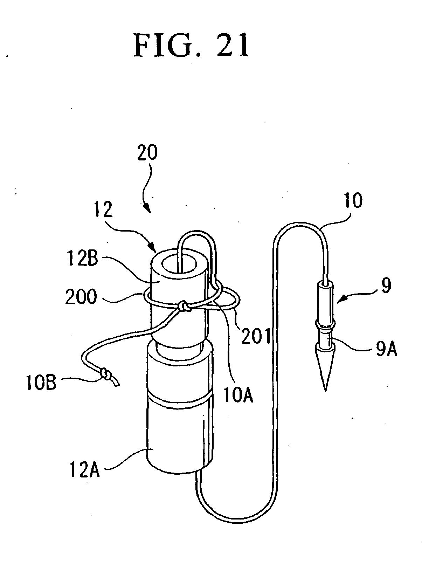

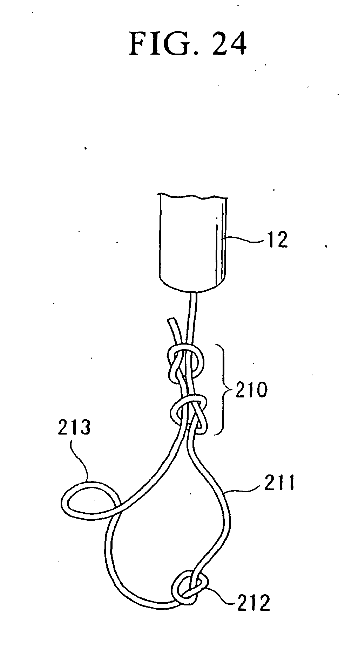

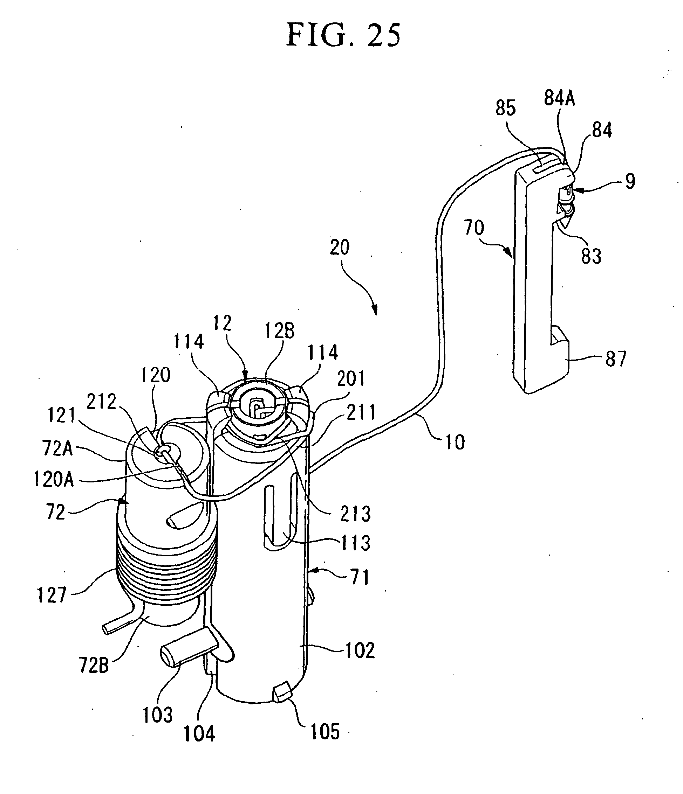

An example modification of an arrangement in which the suture material 10 is wound around the casing 12 is shown in FIGS. 21 through 25.

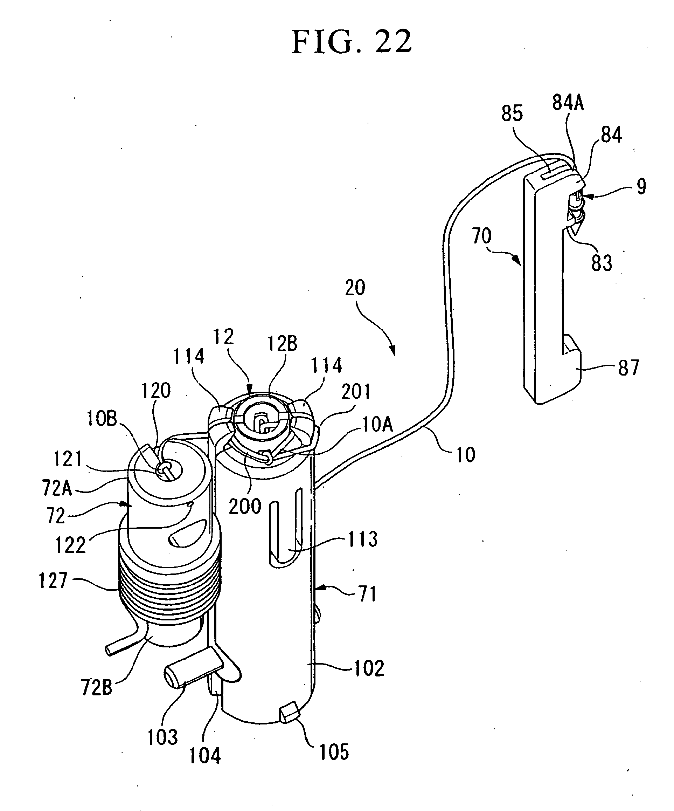

In the pattern shown in FIGS. 21 and 22, the suture material 10 which has been pulled out from the casing 12 is passed through loop 200. The portion 201 passed through loop 200 and pulled out is caught on the outer periphery of the claw 114 of the holder 71. In this case, when pushing the casing 12 with the hook 15, the suture material 10 is pulled by the winding cylinder 72 and the claw 114, so that the tightening force increases. Accordingly, the suture material 10 is even more strongly fixed in place to the hook 15.

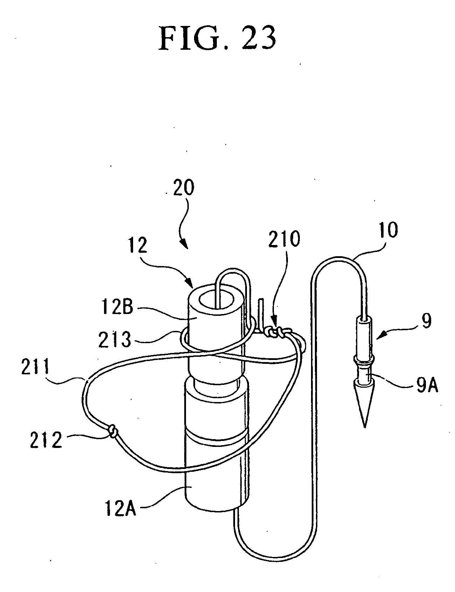

In the pattern shown in FIGS. 23 through 25, a loop 211 is formed using a double slip knot 210. A knot 212 is formed inside the loop 211, and a portion of this loop 211 is then used to form a smaller loop 213. This small loop 213 is wound to casing 12, and the loop 211 is caught on the outer periphery of the claw 114. The knot 212 of the loop 211 is housed inside the center part 121 of winding cylinder 72. In addition to the groove 120, a groove 120A connecting with center part 121 is provided to the winding cylinder 72, and a portion of the loop 211 is caught on the winding cylinder 72. Since the suture material 10 is fastened by being pulled by the winding cylinder 72 and the claw 114 during loading, the tightening force increases and the hook 15 is even more firmly fixed in place.

The present invention is not limited by the above description, but rather is limited only by the appended claims.

For example, the loading device may be altered according to the design of the indwelling implement. Namely, the present invention may be applied to an indwelling implement of the type, such as a detachable snare, in which a loop is formed in advance to the operative region. Traction is applied with the hook 15 to the loop part formed in non-procedure side, and the inner diameter of the snare reduces to tie down on the tissue. In the case of this type of indwelling implement, the same type of action can be obtained by providing a holder for holding the snare, a part equivalent to the casing for winding the loop (i.e., member for forming the wound portion), and a holder for holding the part equivalent to the casing. In the case of an indwelling implement for tying down tissue by tightening a wire with the hook 15, a design results having only the casing holder 50.

This embodiment employs a design having a releasable needle 9, however, the present invention is not limited thereto. Rather, it is also acceptable to provide an end tip other than a needle, and engage this in the casing to form the loop.

## Claims

1. A loading device (30) for an indwelling implement (20) that loads an indwelling implement (20) having a linear member (10) into an applicator (1), the loading device (30) for the indwelling implement (20) comprising:
an end tip holder (40) for holding an end tip (9), said end tip (9) being at one end of the linear member (10) and being freely attachable and releasable with respect to an end tip of a needle (8) of the applicator (1), wherein the end tip holder (40) has a receiving part (61) that guides the end tip of the needle (8) of the applicator (1) onto an end housing (62); and
a casing holder (50) for holding a casing (12), said casing (12) being provided farther to the other end of the linear member (10) than the end tip (9), wherein the axis of the casing holder (50) and the axis of a casing attachment (11) of the applicator (1), which attaches the casing (12), approximately coincide, **characterized by**
a front tip looking member (70) that comes into contact with the end tip (9) Inside the end tip holder (40) so that the axis of the end tip (9) and the axis of the receiving part (61) approximately coincide, the front tip locking member (70) being disposed so as to enable movement between a first position at which the movement of the end tip (9) is restricted, and a second position at which the front tip locking member (70) is removed from the movement path of the needle (8) of the applicator (1) when the needle (8) of the applicator (1) is attached to the end tip (9), wherein
the casing holder (50) and the end tip holder (40) are connected by an arm (60) that is elastically deformable so as to enable relative movement, and wherein the loading device (30) further comprises restricting members (160, 161) provided on the casing holder (50) an on the end tip holder (40), so as to restrict the direction of movement of the end tip holder (40) to the receving direction of attachment of the end tip (9).

2. A loading device according to claim 1, wherein the casing holder (50) comprises a holder (71) provided with a holding member that holds the casing (12) so as to enable pushing in of the casing (12), and a biasing device (72) that pulls the linear member (10) that is attached to the casing (12) held by the holder (71).

3. A loading device according to claim 2, wherein the biasing device (72) comprises a cylindrical body that has a groove (120) that engages with the linear member (10), and a biasing member (127) that biases and rotates the cylindrical body around a rotational axis that is generally parallel to the direction in which the casing (12) is pushed.

4. A loading device according to claim 2, wherein the holder (71) has a pair of holder pieces (101, 102) freely openable and closable, and has an opening and closing controller (105) that opens the holding member when the casing (12) is pulled by the holder pieces (101, 102).

## Patentansprüche

1. Ladevorrichtung (30) für ein Implantat-Gerät (20), die ein Implantat-Gerät (20) mit einem geradlinigen Element (10) in einen Applikator (1) lädt, wobei die Ladevorrichtung (30) für das Implantat-Gerät (20) aufweist:
- einen Endspitzenhalter (40) zum Halten einer Endspitze (9), wobei die Endspitze (9) an einem Ende des geradlinigen Elements (10) bereitgestellt ist und hinsichtlich einer Endspitze einer Nadel (8) des Applikators (1) frei anbringbar und lösbar ist, wobei der Endspitzenhalter (40) ein Aufnahmeteil (61) aufweist, das die Endspitze der Nadel (8) des Applikators (1) in ein Endgehäuse (62) führt; und
- einen Hüllenhalter (50) zum Halten einer Hülle (12), wobei die Hülle (12) weiter bei dem anderen Ende des geradlinigen Elements (10) als die Endspitze (9) angeordnet ist, wobei die Achse des Hüllenhalters (50) und die Achse einer Hüllenaufnahmevorrichtung (11) des Applikators (1), welche die Hülle (12) hält, im Wesentlichen zusammenfallen, **gekennzeichnet durch**
- ein Vorderspitzenverschlusselement (70), das in dem Endspitzenhalter (40) mit der Endspitze (9) in Kontakt tritt, so dass die Achse der Endspitze (9) und die Achse des Aufnahmeteils (61) im Wesentlichen zusammenfallen, wobei das Vorderspitzenverschlusselement (70) derart angeordnet ist, um eine Bewegung zwischen einer ersten Position, bei der die Bewegung der Endspitze (9) eingeschränkt ist, und einer zweiten Position, bei der das Vorderspitzenverschlusselement (70) von dem Bewegungsweg der Nadel (8) des Applikators (1) entfernt ist, wenn die Nadel (8) des Applikators (1) an der Endspitze (9) angebracht ist, ermöglicht, wobei
der Hüllenhalter (50) und der Endspitzenhalter (40) **durch** einen Arm (60) verbunden sind, der elastisch verformbar ist, um eine relative Bewegung zu ermöglichen, und wobei die Ladevorrichtung (30) ferner Beschränkungselemente (160, 161) aufweist, die an dem Hüllenhalter (50) und an dem Endspitzenhalter (40) vorgesehen sind, um die Bewegungsrichtung des Endspitzenhalters (40) in die Aufnahmerichtung der Anbringung der Endspitze (9) einzuschränken.

2. Ladevorrichtung nach Anspruch 1, bei welcher der Hüllenhalter (50) aufweist eine Halterung (71) mit einem die Hülle (12) haltenden Halteelement, um ein Hineindrücken der Hülle (12) zu ermöglichen, und eine Vorspannvorrichtung (72), die das geradlinige Element (10) zieht, das an der durch die Halterung (71) gehaltenen Hülle (12) angebracht ist.

3. Ladevorrichtung nach Anspruch 2, bei welcher die Vorspannvorrichtung (72) aufweist einen zylindrischen Körper mit einer Kerbe (120), die mit dem geradlinigen Element (10) in Eingriff tritt, und ein Vorspannelement (127), das den zylindrischen Körper vorspannt, und den zylindrischen Körper um eine Drehachse dreht, die im Allgemeinen parallel zu der Richtung ist, in welche die Hülle (12) gedrückt wird.

4. Ladevorrichtung nach Anspruch 2, bei welcher die Halterung (71) aufweist ein Paar frei öffenbarer und schließbarer Halterstücke (101, 102), und eine Öffnen- und Schließensteuerung (105), die das Halteelement öffnet, wenn die Hülle (12) von den Haltestücken (101, 102) gezogen wird.

## Revendications

1. Dispositif de chargement (30) pour un instrument à demeure (20) qui charge un instrument à demeure (20) ayant un élément linéaire (10) dans un applicateur (1), le dispositif de chargement (30) pour l'instrument à demeure (20) comprenant :
un porte-pointe d'extrémité (40) pour maintenir une pointe d'extrémité (9), ladite pointe d'extrémité (9) étant prévue à une extrémité de l'élément linéaire (10) et étant librement fixable et libérable par rapport à une pointe d'extrémité d'une aiguille (8) de l'applicateur (1), dans lequel le porte-pointe d'extrémité (40) a une partie de réception (61) qui guide la pointe d'extrémité de l'aiguille (8) de l'applicateur (1) sur un logement d'extrémité (62) ; et
un porte-boîtier (50) pour maintenir un boîtier (12), ledit boîtier (12) étant prévu plus loin vers l'autre extrémité de l'élément linéaire (10) que la pointe d'extrémité (9), dans lequel l'axe du porte-boîtier (50) et l'axe d'une fixation de boîtier (11) de l'applicateur (1), qui fixe le boîtier (12), coïncident approximativement, **caractérisé par**
un élément de verrouillage de pointe avant (70) qui vient en contact avec la pointe d'extrémité (9) à l'intérieur du porte-pointe d'extrémité (40) de sorte que l'axe de la pointe d'extrémité (9) et l'axe de la partie de réception (61) coïncident approximativement, l'élément de verrouillage de pointe avant (70) étant disposé de façon à permettre un mouvement entre une première position à laquelle le mouvement de la pointe d'extrémité (9) est limité, et une deuxième position à laquelle l'élément de verrouillage de pointe avant (70) est retiré du chemin de mouvement de l'aiguille (8) de l'applicateur (1) lorsque l'aiguille (8) de l'applicateur (1) est fixée à la pointe d'extrémité (9), dans lequel
le porte-boîtier (50) et le porte-pointe d'extrémité (40) sont connectés par un bras (60) qui est élastiquement déformable de façon à permettre un mouvement relatif, et dans lequel le dispositif de chargement (30) comprend en outre des éléments de limitation (160, 161) prévus sur le porte-boîtier (50) et sur le porte-pointe d'extrémité (40), de façon à limiter le sens de mouvement du porte-pointe d'extrémité (40) au sens de réception de fixation de la pointe d'extrémité (9).

2. Dispositif de chargement selon la revendication 1, dans lequel le porte-boîtier (50) comprend un dispositif de maintien (71) prévu avec un élément de maintien qui maintient le boîtier (12) de façon à permettre la poussée vers l'intérieur du boîtier (12), et un dispositif d'activation (72) qui tire l'élément linéaire (10) qui est fixé au boîtier (12) maintenu par le dispositif de maintien (71).

3. Dispositif de chargement selon la revendication 2, dans lequel le dispositif d'activation (72) comprend un corps cylindrique qui a une rainure (120) qui s'engage avec l'élément linéaire (10), et un élément d'activation (127) qui active et fait tourner le corps cylindrique autour d'un axe de rotation qui est de façon générale parallèle au sens dans lequel le boîtier (12) est poussé.

4. Dispositif de chargement selon la revendication 2, dans lequel le dispositif de maintien (71) a une paire de parties de maintien (101, 102) librement ouvrables et fermables, et a un contrôleur d'ouverture et de fermeture (105) qui ouvre l'élément de maintien lorsque le boîtier (12) est tiré par les parties de maintien (101, 102).
